# EUROPEAN PATENT APPLICATION

(11) **EP 4 502 941 A1**
(43) Date of publication of application: **05.02.2025**
(21) Application number: 24185117.9
(22) Date of filing: 27.06.2024
(51) Int. Cl.: G06T 7/11

(54) **LUNG REGION SEGMENTATION METHOD AND APPARATUS**

(30) Priority: 03.08.2023 KR 20230101745
(71) Applicant: Medicalip Co., Ltd., Gangwon-do 24341 (KR)
(72) Inventor: PARK, Sang Joon, Seoul (KR); KIM, Jong Min, Yongin-si, Gyeonggi-do (KR); LEE, Da In, Seoul (KR)
(74) Representative: Prendin, Marco

(57) **Abstract**

Provided is a lung region segmentation method and apparatus. The lung region segmentation apparatus extracts a lung region from a two-dimensional (2D) medical image upon input of the 2D medical image, adjusts a size of a mask resembling the lung region, and extracts a peripheral region by removing a region corresponding to the mask from the lung region. The lung region segmentation apparatus further performs a process of extracting a vascular region from the peripheral region.

## Description

### CROSS-REFERENCE TO RELATED APPLICATION

This application is based on and claims priority under 35 U.S.C. §119 to Korean Patent Application No. 10-2023-0101745, filed on August 3, 2023, in the Korean Intellectual Property Office, the disclosure of which is incorporated by reference herein in its entirety.

### BACKGROUND

### 1. Field

The disclosure relates to a method and apparatus for segmenting a lung region, and more particularly, to a method and apparatus for segmenting a peripheral lung region to diagnose a lung disease.

### 2. Description of the Related Art

Medical images such as magnetic resonance imaging (MRI) images, computed tomography (CT) images, X-ray images, etc., are used to diagnose diseases. Three-dimensional (3D) medical images such as MRI images, CT images, etc., may enable accurate diagnosis of lesions by capturing exact images inside human bodies, but require a lot of imaging time or imaging costs. On the other hand, X-ray images may be easily captured, but it is difficult to accurately diagnose lesions using these images due to overlapping between images of several organs. Recently, a method of diagnosing a lesion from a medical image by using an artificial intelligence model has been proposed. 3D medical images such as MRI images, CT images, etc., are mainly used to diagnose lung diseases such as pulmonary hypertension or lung cancer because it is difficult to identify pulmonary hypertension, etc., from X-ray images.

### SUMMARY

Provided is a method and apparatus for segmenting a peripheral lung region to diagnose a lung disease from a two-dimensional (2D) medical image.

Additional aspects will be set forth in part in the description which follows and, in part, will be apparent from the description, or may be learned by practice of the presented embodiments of the disclosure.

According to an aspect of the disclosure, a lung region segmentation method includes receiving a two-dimensional (2D) medical image, extracting a lung region from the 2D medical image, adjusting a size of a mask resembling the lung region, and extracting a peripheral region by removing a region corresponding to the mask from the lung region.

According to another aspect of the disclosure, a lung region segmentation apparatus includes an input unit configured to receive a two-dimensional (2D) medical image, a lung extraction unit configured to extract a lung region from the 2D medical image, a mask adjustment unit configured to adjust a size of a mask resembling the lung region, and a periphery extraction unit configured to extract a peripheral region by removing a region corresponding to the mask from the lung region.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a view showing an example of a lung region segmentation apparatus according to an embodiment of the disclosure;
FIG. 2 is a view showing an example of a lung region segmentation method according to an embodiment of the disclosure;
FIGS. 3 and 4 are views showing an example of a lung region segmentation method according to an embodiment of the disclosure;
FIG. 5 is a view showing an example of a method of generating a mask, according to an embodiment of the disclosure;
FIG. 6 is a view showing an example of a method of adjusting a size of a mask, according to an embodiment of the disclosure;
FIG. 7 shows a vascular region segmented from a peripheral lung region, according to an embodiment of the disclosure;
FIG. 8 shows an example of a ratio of a central lung to a peripheral lung for each lung disease, according to an embodiment of the disclosure; and
FIG. 9 is a block diagram of an example of a lung region segmentation apparatus according to an embodiment of the disclosure.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. Expressions such as "at least one of," when preceding a list of elements, modify the entire list of elements and do not modify the individual elements of the list.

Hereinafter, a lung region segmentation method and apparatus according to an embodiment of the disclosure will be described in detail with reference to the accompanying drawings.

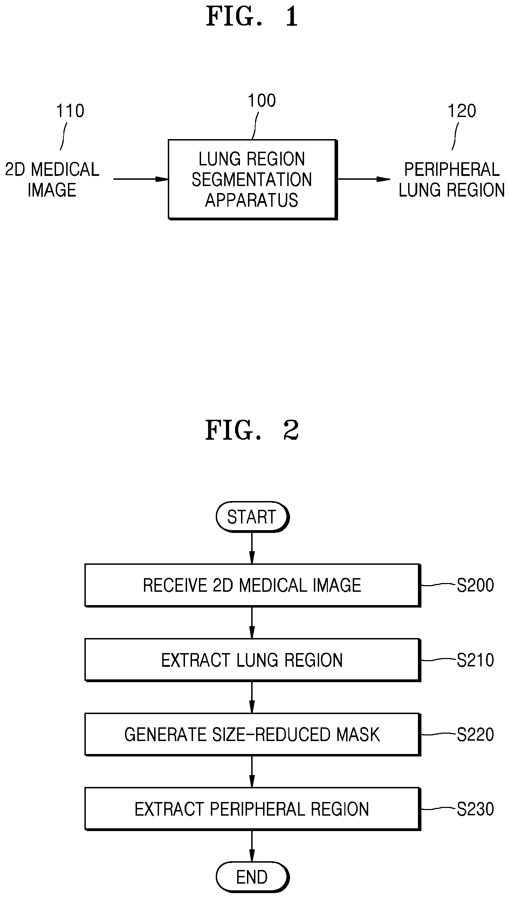

FIG. 1 is a view showing an example of a lung region segmentation apparatus according to an embodiment of the disclosure.

Referring to FIG. 1, a lung region segmentation apparatus 100 may segment a peripheral lung region 120 from a two-dimensional (2D) medical image 110 and output the same. The 2D medical image 110 may be an X-ray image. In addition, the 2D medical image 110 in the current embodiments may be of various types.

The peripheral lung region 120 may be used to diagnose pulmonary hypertension or lung cancer. For example, an area size of blood vessels of the peripheral lung region 120 may have a significant value for diagnosing a lung disease.

FIG. 2 is a view showing an example of a lung region segmentation method according to an embodiment of the disclosure.

Referring to FIG. 2, a lung region segmentation apparatus (hereinafter, referred to as an 'apparatus') may receive a 2D medical image in operation S200. The apparatus may segment a lung region from the 2D medical image in operation S210. The apparatus may use various conventional segmentation algorithms for lung region segmentation.

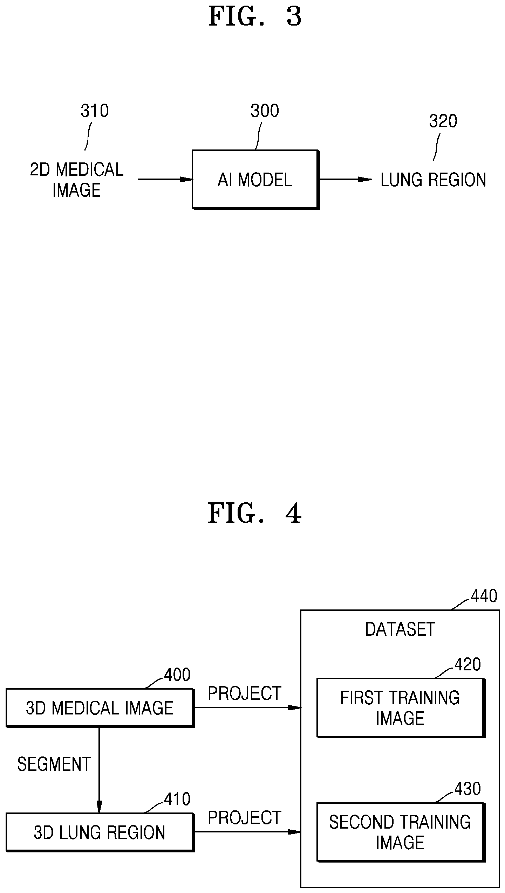

When the 2D medical image is an X-ray image, a plurality of organs are overlappingly displayed. For example, various organs such as ribs, etc., as well as lung tissues may be overlappingly displayed in the lung region segmented from the 2D medical image. From the 2D medical image, only a pure lung region may be segmented without overlapping display of other organs, which will be discussed again with reference to FIGS. 3 and 4.

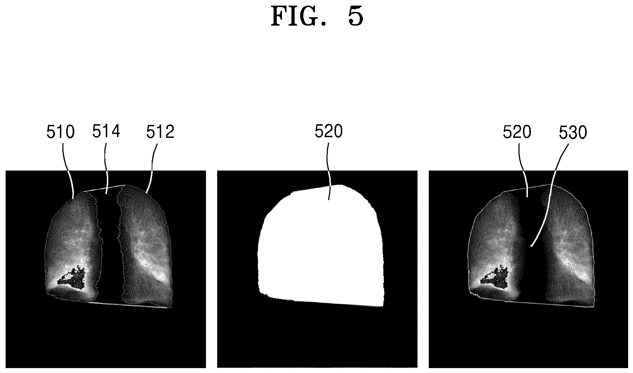

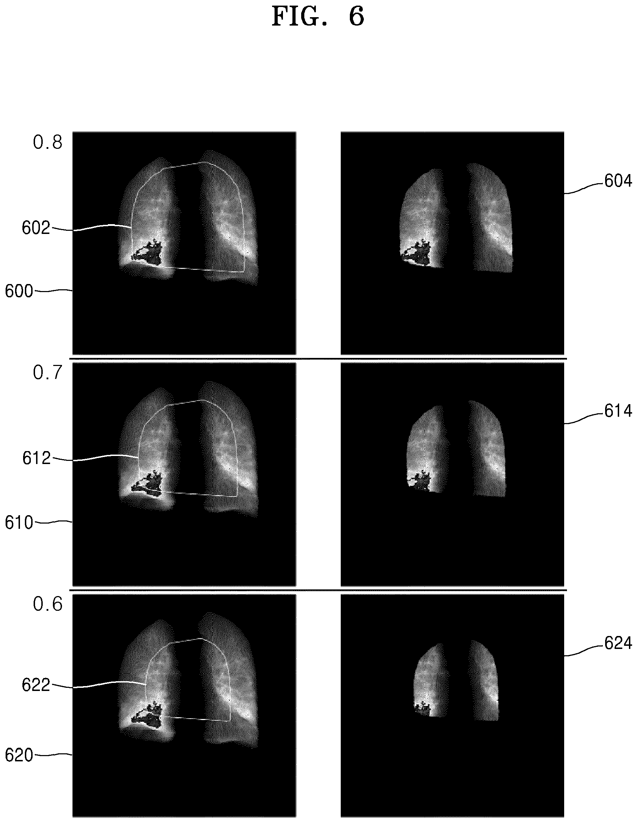

The apparatus may adjust a size of a mask resembling the lung region in operation S220. For example, the apparatus may generate the mask based on an outline of the lung region and reduce the size of the mask. An example of a specific method for generating the mask and adjusting the size thereof will be described again with reference to FIGS. 5 and 6.

The apparatus may extract a peripheral region corresponding to a peripheral lung region from the lung region, by using the size-adjusted mask, in operation S230. For example, the apparatus may remove the mask region from the lung region and output the remaining region as the peripheral lung region. The apparatus may output an image of the extracted peripheral region. By adjusting the size of the mask, the size of the peripheral region extracted from the lung region may be adjusted variously.

The apparatus may further segment a vascular region from the peripheral region. In an embodiment, the apparatus may obtain a ratio of a size of the vascular region (i.e., the entire area of blood vessels) segmented from the entire lung region (or a central lung region) to a size of the vascular region segmented from the peripheral region. In another embodiment, the apparatus may obtain and output a size ratio of the central lung region to the peripheral lung region.

FIGS. 3 and 4 are views showing an example of a lung region segmentation method according to an embodiment of the disclosure.

Referring to FIG. 3, a lung region 320 may be segmented from a 2D medical image 310 by using an artificial intelligence (AI) model 300. The AI model 300 may be implemented as various types of artificial neural networks such as a convolutional neural network, etc. The AI model 300 may be generated to segment the lung region through a training process.

Referring to FIG. 4, an example of the training process of the AI model 300 is shown. A dataset 440 for training the AI model 300 may be generated from a 3D medical image 400. The 3D medical image 400 may be a magnetic resonance imaging (MRI) image or a computed tomography (CT) image.

The apparatus may two-dimensionally project the 3D medical image 400 to generate a 2D first training image 420. For example, each voxel of the 3D medical image 400 may be projected to a 2D plane to generate an image in which a plurality of tissues overlap, like an X-ray image. That is, by two-dimensionally projecting the 3D medical image 400, a virtual X-ray image may be obtained.

The apparatus may segment a 3D lung region 410 from the 3D medical image 400. The apparatus may segment the lung region 410 from the 3D medical image 400 based on a brightness value (i.e., a hounsfield unit (HU)) of a voxel. In addition, conventional various segmentation algorithms for segmenting a human organ from the 3D medical image 400 may be applied to the current embodiment.

The apparatus may two-dimensionally project the 3D lung region 410 to generate a 2D second training image 430. That is, the second training image 430 may be a 2D image including a lung region.

The apparatus may train the AI model 300 by using the dataset 440 including the first training image 420 and the second training image 430. The apparatus may generate the dataset 440 including a plurality of first training images 420 and a plurality of second training images 430 by using a plurality of 3D medical images 400. In an embodiment, the apparatus may label the second training image 430 as ground truth to train the AI model 300 using a supervised training method. The AI model 300 may perform a training process by outputting a predicted image in which the lung region is segmented from the first training image 420 upon input of the first training image 420 of the dataset 440, and comparing the predicted image with the second training image 430 that is the ground truth of the dataset 440 to reduce an error.

FIG. 5 is a view showing an example of a method of generating a mask, according to an embodiment of the disclosure.

Referring to FIG. 5, the apparatus may extract an outline from an image of the lung region segmented from the 2D medical image. In an embodiment, a mask 520 may include a first region 510 including an outline of a left lung and a second region 512 including an outline of a right lung. In another embodiment, the mask 520 may include one enclosed space by connecting an empty space 514 between the first region 510 and the second region 512. For example, the apparatus may connect the highest and lowest height points of the first region 510 to the highest and lowest height points of the second region 512 with line segments, respectively, to fill the empty space 514 between the two regions 510 and 512, thus generating the mask 520 including one enclosed region. Hereinbelow, for convenience, a description will be made based on the mask 520 including one region.

FIG. 6 is a view showing an example of a method of adjusting a size of a mask, according to an embodiment of the disclosure.

Referring to FIGS. 5 and 6, the apparatus may adjust the size of the mask 520 with respect to a center point 530 of the mask 520. For example, examples of adjusting sizes of masks 602, 612, and 622 to 80 %, 70 %, and 60 % are shown in FIG. 6. That is, the apparatus may adjust the sizes of the masks 602, 612, and 622 from lung region images 600, 610, and 620 with respect to the center 530 of the mask 520 while maintaining the shape of the mask 520.

Based on coordinates of the center point 530 of the mask 520 and coordinates of the outline of the mask 520, the apparatus may identify an internally diving point that divides each point of the outline of the mask 520 and a center point 530 at m:n (m and n are natural numbers), and connect the dividing points to generate the size-adjusted masks 602, 612, and 622. Various methods for changing a mask size may be applied to the current embodiment and are not limited to a specific one.

The apparatus may extract, as peripheral lung regions, the other regions than regions of the masks 602, 612, and 622 from the lung region images 600, 610, and 620. The apparatus may also extract, as central lung regions 604, 614, and 624, regions corresponding to masks from lung regions. In FIG. 6, extraction examples of the central lung regions 604, 614, and 624 are shown.

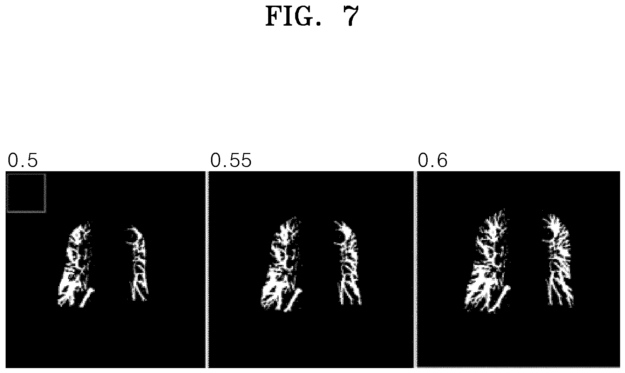

FIG. 7 shows a vascular region segmented from a peripheral lung region, according to an embodiment of the disclosure.

Referring to FIG. 7, the apparatus may extract an edge area of the lung region as a peripheral lung region by adjusting the size of a mask. The apparatus may segment a vascular region from the peripheral lung region. Conventional various segmentation algorithms for segmenting a blood vessel from a human tissue may be applied to the current embodiment. In an embodiment, an AI model trained to segment a blood vessel from a lung tissue may be used in the current embodiment.

In the current embodiment are shown pictures obtained by adjusting a size of a mask to 50 %, 55 %, and 60 % of the original size, extracting a peripheral region from each lung region, and segmenting a vascular region from the peripheral region.

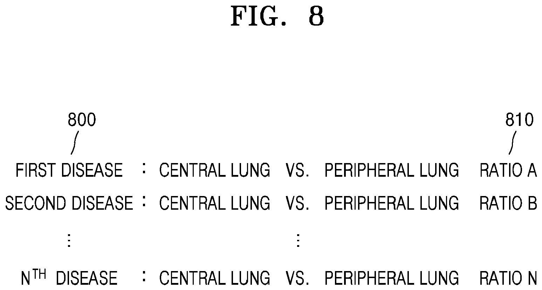

FIG. 8 shows an example of a ratio of a central lung to a peripheral lung for each lung disease, according to an embodiment of the disclosure.

Referring to FIG. 8, an optimal ratio of a central lung to a peripheral lung for disease diagnosis may differ with a type of a lung disease. For example, a ratio 810 of a central lung to a peripheral lung used for diagnosis of a first disease 800 may be 5:5, and a ratio of the central lung to the peripheral lung used for diagnosis of a second disease may be 6:4. That is, to diagnose the first disease, the apparatus may adjust the size of the mask to 50 % of the original size to extract a peripheral lung from the lung region and extract a vascular region of the peripheral lung.

The ratio of the central lung to the peripheral lung for each lung disease in the current embodiment may be identified through statistical analysis of existing clinical information of patients with lung diseases. For example, the apparatus may extract the lung region from the 2D medical image or the 3D medical image of a patient group of the first disease and segment the lung region into the central lung and the peripheral lung at various ratios, thereby identifying a ratio optimized for identifying the corresponding disease.

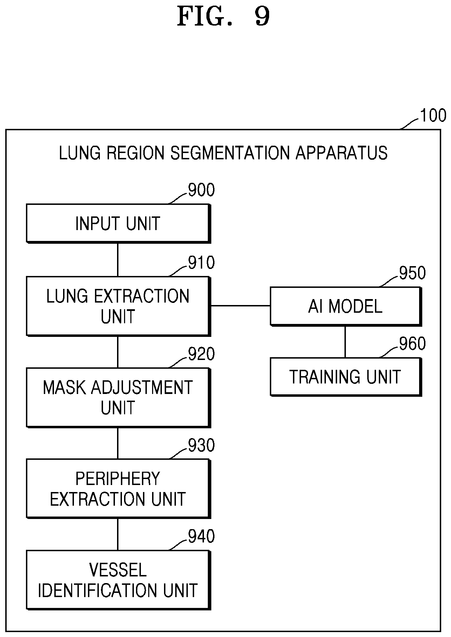

FIG. 9 is a block diagram of an example of a lung region segmentation apparatus according to an embodiment of the disclosure.

Referring to FIG. 9, the lung region segmentation apparatus 100 may include an input unit 900, a lung extraction unit 910, a mask adjustment unit 920, a periphery extraction unit 930, a vessel identification unit 940, an AI model 950, and a training unit 960. In an embodiment, the lung region segmentation apparatus 100 may be implemented as a computing device including a memory, a processor, and an input/output device. In this case, each component may be implemented with software (program) and loaded on the memory, and then may be executed by the processor. In another embodiment, the lung region segmentation apparatus 100 may omit at least one of the vessel identification unit 940, the AI model 950, and the training unit 960.

The input unit 900 may receive a 2D medical image. An example of the 2D medical image may include an X-ray image.

The lung extraction unit 910 may extract a lung region from the 2D medical image. In an embodiment, the lung extraction unit 910 may segment the lung region from the 2D medical image by using an AI model 950. An example of segmenting the lung region by using the AI model 950 is shown in FIG. 3. A training method of the training unit 960 training the AI model 950 is shown in FIG. 4.

The mask adjustment unit 920 may adjust the size of a mask resembling the lung region. For example, the mask adjustment unit 920 may generate the mask including a region formed with the outline of the lung region, and adjust the size of the mask based on the center of the mask. Examples of mask generation and size adjustment are shown in FIGS. 5 and 6.

The periphery extraction unit 930 may extract a peripheral region by removing a region corresponding the mask from the lung region. In another embodiment, the periphery extraction unit 930 may extract the region corresponding to the mask as a central lung region from the lung region. That is, the periphery extraction unit 930 may segment the lung region into the central lung region and the peripheral lung region.

The vessel identification unit 940 may identify a vascular region from the peripheral region. In another embodiment, the vessel identification unit 940 may calculate and output a ratio of the size of the vascular region of the lung region (or the central lung region) to the size of the vascular region of the peripheral region.

The present disclosure may also be implemented as a computer-readable program code on a computer-readable recording medium. The computer-readable recording medium may include all types of recording devices in which data that is readable by a computer system is stored. Examples of the computer-readable recording medium may include read-only memory (ROM), random access memory (RAM), compact-disc ROM (CD-ROM), a magnetic tape, a floppy disk, an optical data storage device, etc. The computer-readable recording medium may be distributed over computer systems connected through a network to store and execute a computer-readable code in a distributed manner.

So far, embodiments have been described for the disclosure. It would be understood by those of ordinary skill in the art that the present disclosure may be implemented in a modified form within a scope without departing from the essential characteristics of the disclosure. Therefore, the disclosed embodiments should be considered in a descriptive sense rather than a restrictive sense. The scope of the present specification is not described above, but in the claims, and all the differences in a range equivalent thereto should be interpreted as being included in the disclosure.

According to an embodiment of the disclosure, a peripheral lung region may be segmented from a 2D medical image such as an X-ray image, etc. The peripheral lung region may be used for accurate diagnosis of a lung disease such as pulmonary hypertension, lung cancer, etc. In another embodiment, a size ratio or a vascular area ratio of a central lung to a peripheral lung may be calculated for each lung disease type.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope of the disclosure as defined by the following claims.

The invention could be inter alia defined by the following examples:
1. A lung region segmentation method comprising:
   receiving a two-dimensional (2D) medical image;
   extracting a lung region from the 2D medical image;
   adjusting a size of a mask resembling the lung region; and
   extracting a peripheral region by removing a region corresponding to the mask from the lung region.
2. The lung region segmentation method of example 1, further comprising extracting a vascular region from the peripheral region.
3. The lung region segmentation method of example 2, further comprising calculating a ratio of a size of a vascular region of the lung region to a size of a vascular region of the peripheral region.
4. The lung region segmentation method of any one of the preceding examples, wherein the 2D medical image comprises an X-ray image.
5. The lung region segmentation method of any one of the preceding examples, wherein the extracting of the lung region comprises extracting the lung region by using an artificial intelligence (AI) model trained to extract the lung region from the 2D medical image.
6. The lung region segmentation method of example 5, wherein the AI model is trained according to a supervised training method by using a dataset comprising a first training image obtained by two-dimensionally projecting a three-dimensional (3D) medical image and a second training image obtained by two-dimensionally projecting a lung region segmented from the 3D medical image.
7. The lung region segmentation method of any one of the preceding examples, wherein the adjusting of the size of the mask comprises:
   extracting an outline from the lung region;
   generating a mask comprising a first region comprising an outline of a left lung, a second region comprising an outline of a right lung, and an empty space between the first region and the second region; and
   reducing the mask by a specific ratio with respect to a central point of the mask.
8. The lung region segmentation method of example 7, wherein the generating of the mask comprises generating the mask comprising one region enclosed by connecting a highest height point and a lowest height point of the first region to a highest height point and a lowest height point of the second region with line segments, respectively.
9. A lung region segmentation apparatus comprising:
   an input unit configured to receive a two-dimensional (2D) medical image;
   a lung extraction unit configured to extract a lung region from the 2D medical image;
   a mask adjustment unit configured to adjust a size of a mask resembling the lung region; and
   a periphery extraction unit configured to extract a peripheral region by removing a region corresponding to the mask from the lung region.
10. The lung region segmentation apparatus of example 9, further comprising a vessel identification unit configured to identify a vascular region from the peripheral region.
11. A computer-readable recording medium having recorded thereon a computer program for executing the lung region segmentation method of any one of examples 1 to 8.

## Claims

1. A lung region segmentation method comprising:
receiving a two-dimensional (2D) medical image;
extracting a lung region from the 2D medical image;
adjusting a size of a mask resembling the lung region; and
extracting a peripheral region by removing a region corresponding to the mask from the lung region.

2. The lung region segmentation method of claim 1, further comprising extracting a vascular region from the peripheral region.

3. The lung region segmentation method of claim 2, further comprising calculating a ratio of a size of a vascular region of the lung region to a size of a vascular region of the peripheral region.

4. The lung region segmentation method of claim 1, wherein the 2D medical image comprises an X-ray image.

5. The lung region segmentation method of claim 1, wherein the extracting of the lung region comprises extracting the lung region by using an artificial intelligence (AI) model trained to extract the lung region from the 2D medical image.

6. The lung region segmentation method of claim 5, wherein the AI model is trained according to a supervised training method by using a dataset comprising a first training image obtained by two-dimensionally projecting a three-dimensional (3D) medical image and a second training image obtained by two-dimensionally projecting a lung region segmented from the 3D medical image.

7. The lung region segmentation method of claim 1, wherein the adjusting of the size of the mask comprises:
extracting an outline from the lung region;
generating a mask comprising a first region comprising an outline of a left lung, a second region comprising an outline of a right lung, and an empty space between the first region and the second region; and
reducing the mask by a specific ratio with respect to a central point of the mask.

8. The lung region segmentation method of claim 7, wherein the generating of the mask comprises generating the mask comprising one region enclosed by connecting a highest height point and a lowest height point of the first region to a highest height point and a lowest height point of the second region with line segments, respectively.

9. A lung region segmentation apparatus comprising:
an input unit configured to receive a two-dimensional (2D) medical image;
a lung extraction unit configured to extract a lung region from the 2D medical image;
a mask adjustment unit configured to adjust a size of a mask resembling the lung region; and
a periphery extraction unit configured to extract a peripheral region by removing a region corresponding to the mask from the lung region.

10. The lung region segmentation apparatus of claim 9, further comprising a vessel identification unit configured to identify a vascular region from the peripheral region.

11. A computer-readable recording medium having recorded thereon a computer program for executing the lung region segmentation method of claim 1.
